# EUROPÄISCHE PATENTSCHRIFT

(11) **EP 3 084 413 B1**
(45) Veröffentlichungstag und Bekanntmachung des Hinweises auf die Patenterteilung: **22.08.2018**
(21) Anmeldenummer: 14821066.9
(22) Anmeldetag: 16.12.2014
(51) Int. Cl.: G01N 27/74, G01N 27/76

(54) **MAGNETOMECHANISCHER SENSOR ZUR PARAMAGNETISCHEN SAUERSTOFFMESSUNG**
MAGNETO-MECHANICAL SENSOR FOR PARAMAGNETIC OXYGEN MEASUREMENT
CAPTEUR MAGNÉTO-MÉCANIQUE POUR LA MESURE PARAMAGNÉTIQUE D'AZOTE

(30) Priorität: 20.12.2013 DE 102013022015
(43) Veröffentlichungstag der Anmeldung: 26.10.2016
(73) Patentinhaber: ABB Schweiz AG, 5400 Baden (CH)
(72) Erfinder: BAUER, Thomas, 61350 Bad Homburg (DE); HARDOCK, Anton, 63071 Offenbach (DE); MÜLLER, Eginhard, 65719 Hofheim (DE); MAURER, Sebastian, 61184 Karben (DE); RAT, Francis, 56235 Ransbach-Baumbach (DE); MARQUART, Nico, 79110 Freiburg (DE)
(74) Vertreter: Marks, Frank
(86) Internationale Anmeldenummer: PCT/EP2014/003367
(87) Internationale Veröffentlichungsnummer: WO 2015/090559

(56) Entgegenhaltungen:
- DE-A1-102006 021 308
- US-A- 5 932 794

## Beschreibung

Die vorliegende Erfindung betrifft einen magnetomechanischen Sensor zur paramagnetischen Gasanalyse mit einem hantelförmigen Probenkörper zur drehbaren Aufhängung in einem inhomogenen Magnetfeld, mindestens einem elektrisch leitenden Spanndraht, der zur drehbaren Aufhängung des Probekörpers koaxial an dessen Drehachse angebracht ist, einer am Probekörper aufgebrachten Leiterschleife, um bei Bestromung in Zusammenwirken mit dem inhomogenen Magnetfeld ein Drehmoment zum Stabilisieren des Probekörpers zu erzeugen sowie einem am Probekörper angeordneten Spiegel als Bestandteil einer optischen Waage, zur Messung einer Auslenkung des Probekörpers.

Das Einsatzgebiet der Erfindung erstreckt sich auf die paramagnetische Gasanalyse, insbesondere der Sauerstoffmessung. Bei dieser Art der Messung wird von der unter den Gasen seltenen Eigenschaft des Sauerstoffs Gebrauch gemacht, dass dieser eine hohe paramagnetische Suszeptibilität besitzt. Liegt in einem Gasgemisch ein inhomogenes magnetisches Feld an, so werden die Sauerstoffmoleküle sich entlang steigender Feldgradienten bewegen und sich dadurch in den Bereichen hoher Feldstärke sammeln. Um dieses Verhalten des Sauerstoffs sichtbar zu machen, wird ein hantelförmiger Probekörper verwendet, der drehbar in einem Gas aufgehängt ist, in dem sich ein geeignetes inhomogenes Magnetfeld befindet. Durch die lokale Verdichtung der paramagnetischen Sauerstoffmoleküle wird ein Drehmoment auf den hantelförmigen Probekörper ausgeübt, der sich infolgedessen in Form einer Drehung um die durch den mindestens einen Spanndraht definierte Achse, vorzugsweise durch den Schwerpunkt des Probekörpers, bewegt. Zur genauen Messung dieser Bewegung wird ein Spiegel als Teil einer Lichtwaage verwendet, die die Abweichung eines Lichtreflexes von diesem Spiegel auf einem Fotosensor misst. Zur Kompensation der Bewegung des Probekörpers wird ein Strom durch die Leiterschleife geleitet, welcher mit dem Magnetfeld wechselwirkt und den Probekörper wieder in seine Ruhelage zurückführt. Die Stärke dieses Stroms ist dann ein Maß für die Sauerstoffkonzentration.

Aus der DE 10 2006 021 308 B4 ist es bekannt, die Leiterschleife auf die Oberfläche des Probekörpers eines paramagnetischen Sauerstoffanalysators aufzudampfen. In diesem Stand der Technik wird der Probekörper durch eine Anordnung von Federn gehalten, die sich planar in einer Ebene mit der Drehebene des Probekörpers befinden. Somit ist es möglich, dass die Leiterschleife nur auf einer Seite, in der Regel der Oberseite, des Probekörpers angebracht ist, und dort galvanisch mit den Federn verbunden ist, die den Strom zur Erzeugung des Drehmoments leiten.

Aus der US 5,932,794 ist ein Sauerstoffsensor bekannt, bei dem der Probenkörper, der Spiegel und die Leiterschleife als mikromechanische Strukturen, insbesondere auf der Basis von Glas oder Silizium ausgebildet sind.

Bei einer Anordnung wie im Oberbegriff dieser Erfindung, in der ein Probekörper durch mindestens einen Spanndraht drehbar gehalten wird, ist diese Konfiguration allerdings nicht möglich, da der Spanndraht sich nicht planar in einer Ebene mit der Drehebene des Probekörpers befindet, sondern weitgehend senkrecht auf dieser Ebene steht. Es muss also eine elektrische Leitung, die auch Teil der Leiterschleife sein kann, von der Oberseite zur Unterseite des Probekörpers existieren, da der Strom hier weiter geleitet wird, um den Stromkreis zu schließen. Die technisch sicherlich naheliegende Aufdampfung einer elektrischen Leitung über eine Kante und über eine dünne Außenfläche hinweg, die nötig wäre, um eine durchgehende Leiterschleife über mehrere Seitenflächen des Probekörpers zu führen, erscheint allerdings unpraktikabel.

Zu den Vorteilen der Aufbringung von elektrischen Leitungen durch Metallisierung statt beispielsweise durch Anlöten von Drähten zählt, dass die Metallisierung einer Oberfläche leicht automatisiert, ohne oder mit wenig manueller, menschlicher Handhabung durchgeführt werden kann, beispielsweise via PVD (Physical Vapor Depostion) oder CVD (Chemical Vapor Deposition). Außerdem ist jede Methode zur Miniaturisierung des Probekörpers wünschenswert, da kleinere, kompaktere und leichtere Probekörper aufgrund ihrer geringen Masse sensibler auf Krafteinwirkungen reagieren. Außerdem können sie in kleineren Kammern von dem zu messenden Gas umspült werden, was die Verzögerung minimiert, mit der ein solcher Sensor auf Änderungen im Sauerstoffgehalt im zu messenden Gas reagiert, da weniger Gas zugeführt werden muss um das umgebende Volumen zu füllen. Durch schnelleres Zuführen von zu messendem Gas ist eine Minimierung dieser Verzögerung nur begrenzt möglich, da der Sensor durch den verbundenen Teilchenfluss ausgelenkt werden kann.

Es ist daher Aufgabe der vorliegenden Erfindung, einen magnetomechanischen Sensor zu schaffen, bei dem die zur galvanischen Verbindung mit einem Spanndraht vorgesehene Leiterschleife mit geringem Fertigungsaufwand herstellbar ist und eine geringe Größe und ein geringes Gewicht besitzt.

Die Aufgabe wird ausgehend von einem Sensor gemäß dem Oberbegriff von Anspruch 1 in Verbindung mit dessen kennzeichnenden Merkmalen gelöst. Die nachfolgenden abhängigen Ansprüche geben vorteilhafte Weiterbildungen der Erfindung wieder.

Die Erfindung schließt die technische Lehre ein, dass zumindest Teile der Leiterschleife durch Metallisierung auf der Oberfläche des Probekörpers aufgetragen sind. Die Metallisierung kann dabei durch verschiedene Dünnschichttechniken realisiert werden, einschließlich Aufdampfen durch PVD, beispielsweise durch Sputtern, und durch CVD.

Vorteilhaft dabei ist, dass so unter anderem geringer Aufwand bei der Produktion sowie eine hohe Sensitivität und geringe Ansprechzeiten des Sensors auf Änderungen beispielsweise des Sauerstoffgehalts im zu messenden Gas erreicht werden können, da der Probekörper mit Leiterschleife mit ein geringem Gewicht und geringen Ausmaßen gefertigt werden kann.

Gemäß einer die Erfindung verbessernden Maßnahme wir vorgeschlagen, dass Durchkontaktierungen im Probekörper vorgesehen sind, die die Leitung von elektrischem Strom von einer Seite des Probekörpers auf eine gegenüberliegende Seite erlauben. Dadurch lässt sich die Leiterschleife in Kombination von einem aufgedampften Teil und mindestens einer Durchkontaktierung vollständig realisieren. Die zu Herstellung benötigten Arbeitsschritte, also das Aufdampfen und die Durchkontaktierung, sind ohne manuelle, menschliche Handhabung realisierbar.

Gemäß einer anderen die Erfindung verbessernden Maßnahme wird vorgeschlagen, auf dem Probekörper im Bereich seines Schwerpunktes eine räumliche Struktur vorzusehen, die einen teilweisen oder vollständigen Formschluss mit einem Endstück von mindestens einem Spanndraht zulässt. Eine solche geeignete Struktur kann dann als Führung bei der Befestigung des Spanndrahtes an dem Probekörper dienen, um den Abstand zu minimieren, den die axiale Achse des Spanndrahts von dem Schwerpunkt des Probekörpers besitzt, und um die manuelle Befestigung des Spanndrahtes am Probekörper zu vereinfachen und um den Zeitaufwand zu verringern, der dazu geleistet werden muss.

Nach einer bevorzugten Ausführungsform der Erfindung wird vorgeschlagen, den Spiegel ebenfalls aufzudampfen, wobei der Spiegel auch als Teil des aufgedampften Teils der Leiterschleife realisiert wird. Dies vereinfacht den Fertigungsprozess.

Zusätzlich kann der Spanndraht je nach Beschaffenheit der zu verbindenden Bauteile durch Löten, durch Verbindung mit leitendem Kleber oder durch Bonding an dem aufgedampften Teil der Leiterschleife angebracht werden. So wird die mechanische und die galvanische Verbindung von Spanndraht und Leiterschleife in einem Arbeitsschritt vollzogen.

In einer bevorzugten Ausführungsform der Erfindung wird vorgeschlagen, dass der Probekörper massiv ausgebildet ist. Zur Fertigung kann der Körper so beispielsweise aus einem Substrat gestanzt oder geschnitten werden. Eine Alternative zu dieser Bauform besteht beispielsweise in einem hohl ausgebildeten Probekörper, welcher beispielsweise mit einem diamagnetischen Gas gefüllt sein kann.

Weitere die Erfindung verbessernde Maßnahmen werden nachstehend gemeinsam mit der Beschreibung eines bevorzugten Ausführungsbeispiels der Erfindung anhand der einzigen Figur näher dargestellt.
- Figur 1: zeigt die Sicht auf die Vorderseite eines erfindungsgemäßen Sensors gemäß einem ersten Ausführungsbeispiel,
- Figur 2: zeigt die Sicht auf dessen Rückseite,
- Figur 3: zeigt einen A-B Schnitt gemäß Figur 1,
- Figur 4: zeigt die Sicht auf die Oberseite eines erfindungsgemäßen Sensors gemäß einem weiteren Ausführungsbeispiel,
- Figur 5: zeigt die Sicht auf dessen Rückseite, und
- Figur 6: zeigt einen A-B Schnitt gemäß Figur 3.

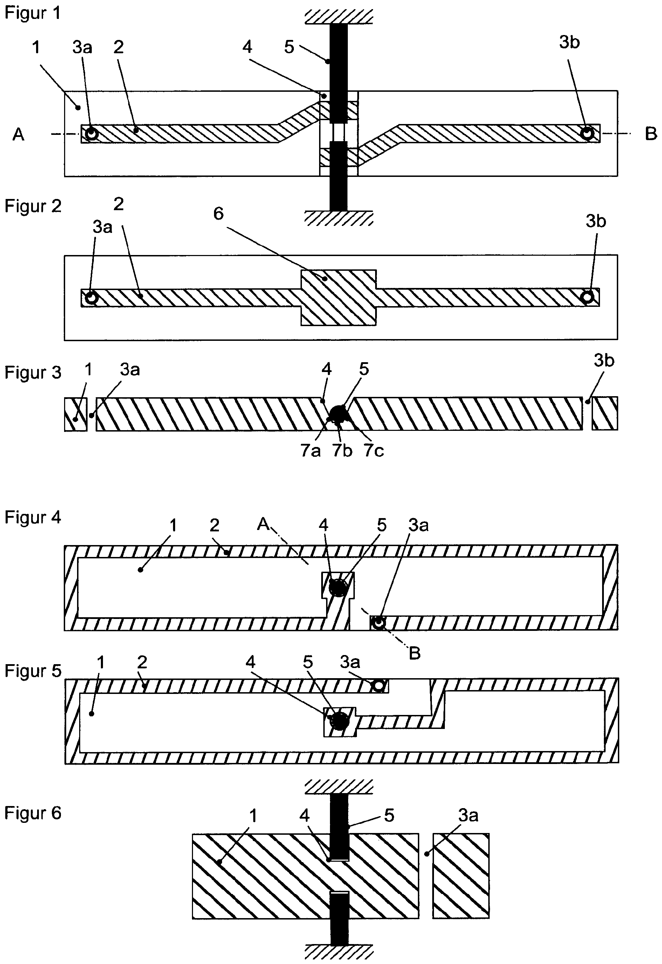

Figur 1 stellt die Vorderseite eines Probekörpers **1** dar, mit einem Teil der aufgedampften Leiterschleife **2,** daran durch nicht dargestelltes Lötzinn befestigtem Spanndraht **5**, einer räumlichen Struktur **4** in teilweisem Formschluss mit einem Endstück des Spanndrahts **5** und die Mündungen zweier Durchkontaktierungen **3a**, **3b**. Der Formschluss ist hier durch eine zylindrische, im Querschnitt trapezförmige Ausnehmung in dem Probekörper **1** realisiert, die bei der manuellen oder maschinellen Positionierung des anliegenden Spanndrahts **5** auf dem Probekörper **1** nur einen Freiheitsgrad in Richtung der Rotationsachse des Probekörpers **1** lässt, wobei dieser Freiheitsgrad für eine koaxiale Befestigung vom Spanndraht **5** am Probekörper **1** unerheblich ist. Somit wird die Genauigkeit der Positionierung des Spanndrahtes **5** erhöht, während der Zeitaufwand für die Positionierung verringert wird.

Figur 2 zeigt die Rückseite des Probekörpers **1,** mit einem weiteren Teil der Leiterschleife **2** und einem Spiegel **6,** der als Teil der Leiterschleife ausgebildet ist. Dieser Teil der Leiterschleife 2 wird durch die beiden Durchkontaktierungen **3a, 3b** mit dem in Figur 1 gezeigten Teil galvanisch verbunden.

Figur 3 zeigt die beiden Durchkontaktierungen **3a, 3b** und den Querschnitt der zylindrischen Ausnehmung. Diese Form ermöglicht einen festen Formschluss und einen galvanischen Kontakt mit einem im Querschnitt kreisförmigen Spanndraht **5** an Berührungslinien **7a, 7b, 7c.** Dies bewirkt eine verbesserte mechanische und galvanische Verbindung gegenüber einer Kontaktierung mit nur einer Berührungslinie, wie sie ohne räumliche Struktur **4** entstehen würde. Gleichzeitig ist ersichtlich, dass der Draht aufgrund der räumlichen Struktur **4** nicht seitlich aus dem Schwerpunkt des Probekörpers heraus bewegt werden kann, was die Anbringung des Drahts bei der Konstruktion des Sensors vereinfacht.

Ein weiteres erfindungsgemäßes Ausführungsbeispiel ist in Figur 4 gezeigt. Es zeigt die Oberseite eines Probekörpers **1** mit einem Spanndraht **5**, der koaxial mit der Drehachse des Probekörpers liegt, eine in Form einer Umwicklung um die Drehachse aufgedampfte Leiterschleife **2,** eine Durchkontaktierung **3a** und eine Bohrung **4** zum Formschluss mit dem Endstück des Spanndrahtes **5.** Die Durchkontaktierung **3a** verbindet die Oberseite des Probekörpers galvanisch mit dessen Unterseite.

In Figur 5 ist die Unterseite abgebildet. Hier zu sehen ist ein weiterer Teil der Leiterschleife **2** in Form einer Umwicklung um die Drehachse des Probekörpers **1,** in gleicher Drehrichtung wie auf der Oberseite, so dass das magnetische Feld, dass durch einen Strom in der Leiterschleife auf der Oberseite erzeugt wird, die gleiche Orientierung besitzt wir das magnetische Feld, das durch den gleichen Strom, der durch die Durchkontaktierung **3a** geleitet wird, auf der Unterseite erzeugt wird. Zusätzlich lässt sich durch Vielschichtbauweise so eine Verstärkung der Kraft erreichen, die durch den Strom in der Leiterschleife **2** verursacht wird. Eine Verminderung der Kraft lässt sich einfach erreichen indem statt Umwicklungen beispielsweise geometrisch gerade Leiterelemente verwendet werden.

In Figur 6 sind die Bohrungen **4** zu sehen, die einen Formschluss mit dem Ende des mindestens einen Spanndrahtes **5** zulassen. Beim Anbringen eines Spanndrahts verbleibt dann kein räumlicher Freiheitsgrad, wodurch die Geschwindigkeit und die erzielte Präzision der manuellen Konstruktion erheblich gesteigert werden können.

Die Erfindung ist nicht beschränkt auf die vorstehend beschriebenen bevorzugten Ausführungsbeispiele. Es sind vielmehr auch Abwandlungen hiervon denkbar, welche vom Schutzbereich der nachfolgenden Ansprüche mit umfasst sind. So ist es beispielsweise auch möglich, dass die Leiterschleife asymmetrisch auf nur einem Arm des hantelförmigen Probekörpers ausgebildet ist. Auch kann die räumliche Struktur zum Formschluss mit einem Endstück des Spanndrahtes in Form einer Erhebung statt einer Ausnehmung realisiert sein.

### Bezugszeichenliste

- 1: Probekörper
- 2: Leiterschleife
- 3a: erste Durchkontaktierung
- 3b: zweite Durchkontaktierung
- 4: räumliche Struktur auf dem Probekörper
- 5: Spanndraht
- 6: Spiegel
- 7a: erste Berührungslinie
- 7b: zweite Berührungslinie
- 7c: dritte Berührungslinie

## Patentansprüche

1. Magnetomechanischer Sensor zur paramagnetischen Gasanalyse mit einem hantelförmigen Probenkörper (1) zur drehbaren Aufhängung in einem inhomogenen Magnetfeld,
- mindestens einem elektrisch leitenden Spanndraht (5), der zur drehbaren Aufhängung des Probekörpers (1) koaxial an dessen Drehachse angebracht ist,
- einer am Probekörper (1) aufgebrachten Leiterschleife (2), um bei Bestromung in Zusammenwirken mit dem inhomogenen Magnetfeld ein Drehmoment zum Stabilisieren des Probekörpers (1) zu erzeugen,
- einem am Probekörper (1) angeordneten Spiegel (6) als Bestandteil einer optischen Waage, zur Messung einer Auslenkung des Probekörpers (1),
- wobei zumindest Teile der mit dem Spanndraht (5) galvanisch verbundenen Leiterschleife (2) durch Metallisierung auf der Oberfläche des Probekörpers (1) aufgetragen sind,
**dadurch gekennzeichnet,**
**dass** der Spiegel (6) durch Metallisierung auf den Probekörper (1) aufgetragen ist und dass der Spiegel (6) ein Tei der Leiterschleife (2) ist

2. Vorrichtung nach Anspruch 1,
**dadurch gekennzeichnet,**
**dass** zumindest ein Teil der Leiterschleife (2) in Form mindestens einer Durchkontaktierung (3a) ausgebildet ist, um einen galvanischen Kontakt von Teilen der Leiterschleife (2) auf parallelversetzten Flächen zueinander herzustellen.

3. Vorrichtung nach Anspruch 1,
**dadurch gekennzeichnet,**
**dass** auf dem Probekörper (1) mindestens eine Struktur (4) ausgeprägt ist, die einen Formschluss mit einem Endstück mindestens eines Spanndrahts (5) zulässt, um die Befestigung des Spanndrahtes (5) zu vereinfachen.

4. Vorrichtung nach einem der Ansprüche 1-3,
**dadurch gekennzeichnet,**
**dass** der mindestens eine Spanndraht (5) durch Löten auf der Leiterschleife (2) angebracht ist.

5. Vorrichtung nach einem der Ansprüche 1-3,
**dadurch gekennzeichnet,**
**dass** der mindestens eine Spanndraht (5) durch Verwendung von Leitkleber auf der Leiterschleife (2) angebracht ist.

6. Vorrichtung nach einem der Ansprüche 1-3,
**dadurch gekennzeichnet,**
**dass** der mindestens eine Spanndraht (5) durch Bonding auf der Leiterschleife (2) angebracht ist.

7. Vorrichtung nach einem der vorherigen Ansprüche,
**dadurch gekennzeichnet,**
**dass** der Probekörper (1) massiv ausgebildet ist.

## Claims

1. Magnetomechanical sensor for paramagnetic gas analysis having a dumbbell-shaped test specimen (1) for being rotatably suspended in an inhomogeneous magnetic field,
- at least one electrically conductive tensioning wire (5) which, for rotatably suspending the test specimen (1), is fitted coaxially to the rotation axis of the said test specimen,
- a conductor loop (2), which is attached to the test specimen (1), in order to generate a torque for stabilizing the test specimen (1) in interaction with the inhomogeneous magnetic field when current is supplied,
- a mirror (6), which is arranged on the test specimen (1), as a constituent part of a set of optical scales, for measuring a deflection of the test specimen (1),
- wherein at least parts of the conductor loop (2) which are electrically conductively connected to the tensioning wire (5) are applied to the surface of the test specimen (1) by metallization,
**characterized**
**in that** the mirror (6) is applied to the test specimen (1) by metallization, and in that the mirror (6) is a part of the conductor loop (2).

2. Apparatus according to Claim 1,
**characterized**
**in that** at least one part of the conductor loop (2) is designed in the form of at least one plated through-hole (3a) in order to establish mutual electrically conductive contact of parts of the conductor loop (2) on surfaces which are offset in parallel.

3. Apparatus according to Claim 1,
**characterized**
**in that** at least one structure (4) is stamped on the test specimen (1), the said structure permitting an interlocking connection with an end piece of at least one tensioning wire (5) in order to simplify fastening of the tensioning wire (5).

4. Apparatus according to one of Claims 1-3,
**characterized**
**in that** the at least one tensioning wire (5) is fitted on the conductor loop (2) by soldering.

5. Apparatus according to one of Claims 1-3,
**characterized**
**in that** the at least one tensioning wire (5) is fitted on the conductor loop (2) by using conductive adhesive.

6. Apparatus according to one of Claims 1-3,
**characterized**
**in that** the at least one tensioning wire (5) is fitted on the conductor loop (2) by bonding.

7. Apparatus according to one of the preceding claims,
**characterized**
**in that** the test specimen (1) is of solid design.

## Revendications

1. Capteur magnéto-mécanique pour l'analyse paramagnétique de gaz, comportant un corps échantillon en forme d'haltère (1) destiné à être suspendu en rotation dans un champ magnétique non homogène,
- au moins un fil de tension électriquement conducteur (5) installé de manière à suspendre en rotation le corps échantillon (1) coaxialement à son axe de rotation,
- une boucle conductrice (2) installée sur le corps échantillon (1) afin de générer un couple destiné à stabiliser le corps échantillon (1) lors de l'alimentation en courant en association avec le champ magnétique non homogène,
- un miroir (6) disposé sur le corps échantillon (1) en tant que composant d'une balance optique, pour mesurer une déviation du corps échantillon (1),
- dans lequel au moins certaines parties de la boucle conductrice (2) reliées galvaniquement au fil de tension (5) sont appliquées par métallisation sur la surface du corps échantillon (1),
**caractérisé en ce que** le miroir (6) est appliqué par métallisation sur le corps échantillon (1) et **en ce que** le miroir (6) est une boucle conductrice (2).

2. Dispositif selon la revendication 1,
**caractérisé en ce qu'**au moins une partie de la boucle conductrice (2) est réalisée sous la forme d'au moins une métallisation traversante (3a) afin d'établir un contact galvanique entre des parties de la boucle conductrice (2) sur des surfaces décalées parallèlement.

3. Dispositif selon la revendication 1, **caractérisé par** la réalisation sur le corps échantillon (1) d'au moins une structure (4) permettant un ajustement par complémentarité de forme avec une pièce d'extrémité d'au moins un fil de tension (5) afin de simplifier la fixation du fil de tension (5).

4. Dispositif selon l'une des revendications 1-3, **caractérisé en ce que** l'au moins un fil de tension (5) est fixé à la boucle conductrice (2) par brasage.

5. Dispositif selon l'une des revendications 1-3, **caractérisé en ce que** l'au moins un fil de tension (5) est installé sur la boucle conductrice (2) en utilisant un adhésif conducteur.

6. Dispositif selon l'une des revendications 1-3, **caractérisé en ce que** l'au moins un fil de tension (5) est installé sur la boucle conductrice (2) par collage.

7. Dispositif selon l'une des revendications précédentes, **caractérisé en ce que** le corps échantillon (1) présente une structure pleine.
